# EUROPEAN PATENT SPECIFICATION

(11) **EP 2 187 835 B1**
(45) Date of publication and mention of the grant of the patent: **05.10.2016**
(21) Application number: 08795733.8
(22) Date of filing: 29.08.2008
(51) Int. Cl.: A61C 13/00, A61C 13/107, A61C 13/15, A61K 6/083, A61K 6/09, A61K 6/087

(54) **THREE-DIMENSIONAL PRINTING METHODS AND MATERIALS FOR MAKING DENTAL PRODUCTS**
DREIDIMENSIONALE DRUCKTECHNIK-VERFAHREN UND MATERIALIEN ZUR HERSTELLUNG ZAHNÄRZTLICHER PRODUKTEN
PROCÉDÉS D'IMPRESSION EN TROIS DIMENSIONS ET MATÉRIAUX POUR FABRIQUER DES PRODUITS DENTAIRES

(30) Priority: 31.08.2007 US 967066 P
(43) Date of publication of application: 26.05.2010
(73) Proprietor: DENTSPLY International Inc., York, PA 17405-0872 (US)
(72) Inventor: SUN, Benjamin, J., York, PA 17402 (US); KENNEDY, Christopher, R., New Freedom, PA 17349-9169 (US); LICHKUS, Andrew, York, PA 17404 (US)
(74) Representative: Hartz, Nikolai
(86) International application number: PCT/US2008/010296
(87) International publication number: WO 2009/032228

(56) References cited:
- EP-A1- 1 240 878
- US-A- 6 057 383
- US-B2- 7 189 344

## Description

### CROSS-REFERENCE TO RELATED APPLICATIONS

This application claims the benefit of U.S. Provisional Patent Application No. 60/967,066 having a filing date of August 31, 2007.

### BACKGROUND OF THE INVENTION

### Field of the Invention

The present invention relates generally to rapid prototyping systems for making dental devices and prostheses such as, for example, artificial teeth, dentures, splints, veneers, inlays, onlays, copings, frame patterns, crowns and bridges and the like. More particularly, ink-jet printing systems are used to deposit material on a layer-by-layer basis to build-up the dental device and prosthesis as a three-dimensional object.

### Brief Description of the Related Art

In general, rapid prototyping refers to a conventional manufacturing process used to make parts, wherein the part is built on a layer-by-layer basis using layers of hardening material. Per this technology, the part to be manufactured is considered a series of discrete cross-sectional regions which, when combined together, make-up a three-dimensional structure. The building-up of a part layer-by-layer is very different than conventional machining technologies, where metal or plastic pieces are cut and drilled to a desired shape. In rapid prototyping technology, the parts are produced directly from computer-aided design (CAD) or other digital images. Software is used to slice the digital image into thin cross-sectional layers. Then, the part is constructed by placing layers of plastic or other hardening material on top of each other. There are many different techniques that can be used to combine the layers of structural material. A curing step may be required to fully cure the layers of material.

Ink-jet printing technology is a rapid prototyping method that can be used to fabricate the three-dimensional object. In one well known ink-jet printing method that was developed at Massachusetts Institute of Technology, as described in Sachs et al., US Patent 5,204,055, printer heads are used to discharge a binder material onto a layer of powder particulate in a powder bed. The powdered layer corresponds to a digitally superposed section of the object that will be produced. The binder causes the powder particles to fuse together in selected areas. This results in a fused cross-sectional segment of the object being formed on the platform. The steps are repeated for each new layer until the desired object is achieved. In a final step, a laser beam scans the object causing the powdered layers to sinter and fuse together. In another ink-jet printing process, as described in Sanders, US Patents 5,506,607 and 5,740,051, a low-melting thermoplastic material is dispensed through one ink-jet printing head to form a three-dimensional object. A second ink-jet printer head dispenses wax material to form supports for the three-dimensional object. After the object has been produced, the wax supports are removed, and the object is finished as needed.

Leyden et al., US Patents 6,660,209 and 6,270,335 disclose an ink-jet printing method using commercial print heads having multiple orifices (jets) to selectively fire droplets of hot melt, radiation-curable material onto a substrate. Each orifice can be equipped with a piezoelectric element that causes a pressure wave to propagate through the material when electric current is applied. The print head moves along a scan path selectively depositing the flowable material onto the substrate. In a subsequent step, light radiation is used to cure the material.

Yamane et al., US Patent 5,059,266 discloses an ink-jetting method, whereby a photosetting or thermosetting resin is jetted along a flight passage of the material to a stage to thereby laminate the material on the stage, changing at least one of a jetting direction of the material along the flight passage and a jetting amount of the material, thereby controlling a jetting operation of the material, and exposing the laminated material to light to cure the material, thereby forming the article.

Bredt et al., US Patent 5,902,441 describes another ink-jet printing method, which involves applying a layer of powder particles containing an activatable adhesive onto a flat surface that can be indexed downward. The ink-jet printer introduces an activating fluid onto to the layer of particles in a predetermined pattern. The fluid activates the adhesive in the mixture, causing the particles to adhere together in an essentially solid layer. After the first cross-sectional portion of the article is formed, the movable surface can be indexed downward. Successive layers of the mixture of particles are applied in the same manner to form the desired article.

Oriakhi et al., US Patent Application Publication No. US 2005/0082710 discloses an ink-jet printing method, wherein a particulate blend of reactive glass ionomer particulates, cross-linkable polyacid particulates including polyvinyl pyrrolidone-co-polyacrylic acid, and nanocomposites is spread in a fabrication bin. An ink-jet printer applies an aqueous phase binder onto a predetermined area of the particulate blend to form hydrated cement. A glass-ionomer chemical reaction causes the hydrated cement to harden.

Kapserchik et al., US Patent Application Publication No. US 2004/0094058 discloses an ink-jet printing system using acid-base cements. Layers of powder particulate are deposited on a flat surface. The powders include a base such as a metal oxide or an aluminosilicate glass, a polymeric acid or other acid. The ink-jet printer dispenses an aqueous binder. The basic powder interacts with the acid in the presence of water, causing the formation of an ionically cross-linked hydrogel salt. Formation of the cross-linked hydrogel causes setting of the mixture.

More particularly, ink-jet printing methods for making three-dimensional dental products have been developed and are described in the patent literature.

For example, Moszner et al., US Patent 6,939,489 discloses a process for fabricating three-dimensional dental form pieces for dental restoration and replacement parts using three-dimensional plotting technology. The object is produced in a layered manner by the cutting away of micro drops or micro cords discharged from nozzles in the three-dimensional plotter. The discharged material can be hardened by a variety of mechanisms depending upon the type of material used. This includes cooling of melted material, polycondensation, polyaddition, or thermal-curing, and light radiation. In the '489 Patent, the three-dimensional plotting technology is described as being different than conventional rapid prototyping (selective laser sintering, 3D printing, and stereolithography).

Rheinberger et al., US Patent 7,189,344 discloses a process for producing three-dimensional dental restorative parts, such as full or partial dental prosthesis, using ink-jet printers that are used in the ink-jet printing methods developed by MIT as described above. The process involves spraying a polymerizable material onto a base support in a layer-by-layer manner. Each layer of material is polymerized by a light source prior to the application of the next layer. The polymerizable material is described as being wax-like having up to 70% by weight of at least one of a polymerizable monomer and oligomer; from 0.01 to 10% by weight of a polymerization initiator; and at least 20% by weight of a mixture having a selected one of a wax-like and flowable monomer and a color pigment.

Feenstra, US Patents 6, 921,500 and 6,955,776 disclose an ink-jet printing process for making dental elements such as crowns using a liquid binder and powder bed. The element is produced by applying successive layers of powder and discharging the liquid binder onto the layers using an ink-jet printer. The binder preferably includes nanomeric, inorganic solid particles having polymerizable and/or polycondensable organic groups at their surface. After the binder has been applied to the last layer of powder, any excess, unbound powder is removed. Then, the powdered layers are sintered by heating to a temperature in the range of about 400 to 800°C. The sintering step is performed so that only necks between the powder particles are formed. The resulting sintered dental element is infiltrated by a second phase material, such as glass-ceramic or polymer, which melts at a lower temperature than the material of the dental element. This reduces the porosity of the dental element.

Bordkin et al., US Patent 6,322,728 discloses an ink-jet printing process for making dental restorations by printing a binder into layers of powder. The process involves depositing a layer of ceramic or composite powder material onto a powder bed. The design of the restoration is based on a CAD representation. A binding material is applied onto the ceramic or composite layer. This application of powder/binder material is repeated several times to produce the desired shape of the restoration. After the layering process is completed, the structure is cured to further promote binding of the particles.

US 6 057 383 discloses a method for making a three-dimensional dental prosthesis, comprising the steps of: applying wax-like polymerizable material onto a support surface to form an uncured prosthesis; placing the prosthesis in the mouth of a patient and shaping the prosthesis over a targeted area by applying sufficient pressure; and irradiating the prosthesis with curing light while still in the mouth so that the prosthesis fully cures and hardens.

The present invention provides several different ink-jet printing methods for fabricating three-dimensional dental devices and prostheses. Although the ink-jet printing methods are described primarily herein as being used to make a denture, it should be understood that this is for illustration purposes only. The ink-jet printing methods can be used to make any dental device and prosthesis. By the term, "dental device" or "prosthesis" as used herein, it is meant any product that replaces or restores lost tooth structure, teeth, or oral tissue including, but not limited to, crown and bridges, fillings, inlays, onlays, veneers, restorations, baseplates, splints, denture liners, artificial teeth, copings, frame patterns, full and partial dentures, temporary and permanent dentures, and the like.

### SUMMARY OF THE INVENTION

In a first ink-jet printing method of the invention, a wax-like, polymerizable material is deposited onto a working platform or support surface to form a first cross-sectional layer of the dental device. A second layer of material is then applied over the first layer. Successive layers of wax-like, polymerizable material are added in this manner until the device is completely fabricated. Then, the device, for example, a denture, is placed inside of the mouth and positioned over the patient's upper and/or lower dental arches. The patient bites down upon the denture so that the margins, contacts, and occlusion can be checked by the dentist. This process is referred to as "trying-in" the denture. After completing the try-in step, the dentist makes any necessary adjustments, tries in the denture a second time, and finishes the denture so that it is ready for final curing. In the light-curing step, the denture is exposed to light radiation so that each layer of wax-like, polymerizable material is cured. The fully cured and finished denture is now ready to be used by the patient.

In a second method, an ink-jet printer discharges successive layers of wax-like, polymerizable material onto a platform to form the denture. Then, the shaped device is partially cured by exposing it to light irradiation. The device does not fully harden but maintains some flexibility. The partially cured denture can be placed in the mouth as a try-in. Because the denture is only partially cured at this point, some adjustments can be made. Once the denture has been properly fitted, it is fully cured by treating it with light irradiation. The fully cured denture is finished and trimmed so that it is ready for wear by the patient.

In a fourth method, an ink-jet printing head discharges a "binder' material onto selected areas of a layer of powder particulate in a powder bed. The binder fuses the powder particulate together to form the first cross-sectional layer. The bound powder particulate in the first layer is supported by unbound powder. A second layer of powder is prepared and the binder material is discharged into this layer to form the second cross-sectional layer. Once each layer has been prepared, the product can be removed from the unbound powder and then cured to form the finished product. The dental practitioner can place both the uncured and cured dentures in a patient's mouth as a "try-in" so that the comfort and fit of the device can be checked.

### DETAILED DESCRIPTION OF THE INVENTION

In the present invention, a wax-like polymerizable material is used to manufacture the dental device. This material has high mechanical strength and integrity when it is in an uncured condition. Further, the material has good biocompatibility making it ideal for dental applications. The wax-like polymerizable material can be prepared using the following components.

### Wax-Like Polymerizable Materials

A wax-like, polymerizable material is used to make the dental products in accordance with the methods of this invention. By the term, "wax-like" as used herein, it is meant a material which is flowable (fluid) at a temperature of 40°C and greater, and solidifies (becomes non-fluid) at a temperature of less than or equal to 23°C within 5 minutes of being held at that temperature.

When the wax-like material is at a temperature in the range of 40°C to 140°C, it becomes dimensionally stable within 5 minutes by cooling it to a temperature in the range of 0°C to 37°C. Flowable wax-like material having a temperature in the range of 40°C to 140°C, becomes dimensionally stable within (in order of increasing preference) 4, 2, 1 or 0.5 minutes by cooling it to a temperature in the range of 0°C to 23°C. The following components can be used to prepare the wax-like polymerizable material in accordance with this invention.

### Polymerizable Acrylic Compounds

Polymerizable acrylic compounds that can be used in the compositions of this invention, include, but are not limited to, mono-, di- or poly-acrylates and methacrylates such as methyl acrylate, methyl methacrylate, ethyl acrylate, isopropyl methacrylate, n-hexyl acrylate, stearyl acrylate, allyl acrylate, stearyl methacrylate, the reaction product of octadecyl isocyanate and 2-hydroxyethyl methacrylate, the reaction product of octadecyl isocyanate and caprolactone 2-(methacryloyloxy)ethyl ester, the reaction product of octadecyl isocyanate and 2-hydroxyethyl acrylate; the reaction product of octadecyl isocyanate and hydroxypropyl (meth)acrylate; the reaction product of octadecyl isocyanate and 2-hydroxypropyl 2-(methacryloyloxy)-ethyl phthalate; the reaction product of octadecyl isocyanate and 2-hydroxy-3-phenoxypropyl acrylate; the reaction product of octadecyl isocyanate and glycerol dimethacrylate; the reaction product of octadecyl isocyanate and pentaerythritol triacrylate; the reaction product of cyclohexyl isocyanate and 2-hydroxyethyl (meth)acrylate; the reaction product of benzyl isocyanate and 2-hydroxyethyl (meth)acrylate; 1,14-tetradecanedimethacrylate, dimethylol tricyclodecane diacrylate, glycerol diacrylate, glycerol triacrylate, ethyleneglycol diacrylate, diethyleneglycol diacrylate, triethyleneglycol dimethacrylate, tetraethylene glycol di(meth)acrylate, 1,3-propanediol diacrylate, 1,3-propanediol dimethacrylate, trimethylolpropane tri(meth)acrylate, 1,2,4-butanetriol trimethacrylate, 1,4-cyclohexanediol diacrylate, 1,4-cyclohexanediol dimethacrylate, 1,6-hexanediol di(meth)acrylate, pentaerythritol triacrylate, pentaerythritol tetraacrylate, pentaerythritol tetramethacrylate, sorbitol hexacrylate, 2,2-bis[4-(2-hydroxy-3-acryloyloxypropoxy)phenyl]propane; 2,2-bis[4-(2-hydroxy-3-methacryloyloxypropoxy)phenyl]propane (Bis-GMA); the reaction product of Bis-GMA and octadecyl isocyanate; the reaction product of Bis-GMA and cyclohexyl isocyanate; 2,2-bis[4-(acryloyloxy-ethoxy)phenyl]propane; 2,2-bis[4-(methacryloyloxy-ethoxy)phenyl]propane (or ethoxylated bisphenol A-dimethacrylate) (EBPADMA); urethane di(meth)acrylate (UDMA), diurethane dimethacrylate (DUDMA), 4,13-dioxo-3,14 dioxa-5,12-diazahexadecane-1,16-diol diacrylate; 4,13-dioxo-3,14 dioxa-5,12-diazahexadecane-1,16-diol dimethacrylate; 4,19-dioxo-3,20 dioxa-5,18-diazahexadecane-1,22-diol diacrylate; 4,19-dioxo-3,20 dioxa-5,18-diazahexadecane-1,22-diol dimethacrylate; the reaction product of trimethyl 1,6-diisocyanatohexane and bisphenol A propoxylate and 2-hydroxyethyl methacrylate (TBDMA); the reaction product of 1,6 diisocyanatohexane and 2-hydroxyethyl methacrylate modified with water (HDIDMA); the reaction product of 1,6 diisocyanatohexane and 2-hydroxyethyl acrylate modified with water (HDIDA); the reaction product of 1,6-diisocyanatohexane, 1,2-decanediol, 1,10-decanediol and 2-hydroxyethyl (meth)acrylate; the reaction product of 1,6-diisocyanatohexane, 3-hydroxy 2,2-dimethylpropyl 3-hydroxy-2,2-dimethyl propionate, 1,10-decanediol and 2-hydroxyethyl (meth)acrylate; the reaction product of 1,6-diisocyanatohexane, 1,10-decanediol and 2-hydroxyethyl (meth)acrylate; the reaction product of 1,6-diisocyanatohexane, 1,2-decanediol, 1,10-decanediol, 3-hydroxy 2,2-dimethylpropyl 3-hydroxy-2,2-dimethyl propionate and 2-hydroxyethyl (meth)acrylate; the reaction product of 1,6-diisocyanatohexane, trimethyl 1,6-diisocyanatohexane, 1,10-decanediol and 2-hydroxyethyl (meth)acrylate; the reaction product of 1,6-diisocyanatohexane, trimethyl 1,6-diisocyanatohexane, 3-hydroxy 2,2-dimethylpropyl 3-hydroxy-2,2-dimethyl propionate, 1,10-decanediol and 2-hydroxyethyl (meth)acrylate; the reaction product of 1,6-diisocyanatohexane, 2,5-dimethyl-2,5-hexanediol and 2-hydroxyethyl (meth)acrylate; the reaction product of 1,6-diisocyanatohexane, 4,4'-isopropylidenedicyclohexanol and 2-hydroxyethyl (meth)acrylate; the reaction product of 1,6-diisocyanatohexane, 1,2-decanediol, 1,10-decanediol, 3-hydroxy 2,2-dimethylpropyl 3-hydroxy-2,2-dimethyl propionate and 2-hydroxyethyl (meth)acrylate; the reaction products of 2-isocyanatoethyl methacrylate and diols; the reaction product of 1,6-diisocyanatohexane, 1,2-decanediol, 1,10-decanediol, 1,12-dodecaneediol, 1,6-hexanediol, and 2-hydroxyethyl(meth)acrylate); polyurethane dimethacrylate (PUDMA); alkoxylated pentacrythritol tetraacrylate; polycarbonate dimethacrylate (PCDMA); the bis-acrylates and bis-methacrylates of polyethylene glycols; and copolymerizable mixtures of acrylated monomers and acrylated oligomers, and the like..

### Polymerization System

Ink jet printable polymerizable dental materials and compositions of this invention may include one or more initiating systems to cause them to harden promptly. Light curable wax-like polymerizable dental composites preferably include a light sensitizer, for example camphorquinone, 2,4,6- trimethylbenzoyldiphenylphosphine oxide, or methyl benzoin which causes polymerization to be initiated upon exposure to activating wavelengths of light; and/or a reducing compound, for example tertiary amine.

In one embodiment, a photoactive agent such as, for example, benzophenone, benzoin and their derivatives, or alpha-diketones and their derivatives is added to the composition in order to make it light-curable. A preferred photopolymerization initiator is camphorquinone (CQ). Photopolymerization can be initiated by irradiating the composition with blue, visible light preferably having a wavelength in the range of about 400 to about 500 nm. A standard dental blue light-curing unit can be used to irradiate the composition. The camphorquinone (CQ) compounds have a light absorbency maximum of between about 400 to about 500 nm and generate free radicals for polymerization when irradiated with light having a wavelength in this range. Photoinitiators selected from the class of acylphosphine oxides can also be used. These compounds include, for example, monoacyl phosphine oxide derivatives, bisacyl phosphine oxide derivatives, and triacyl phosphine oxide derivatives. For example, 2,4,6-trimethylbenzoyl-diphenylphosphine oxide (TPO) can be used as the photopolymerization initiator.

In addition to the photoactive and heat activated agents, the material of this invention may include a polymerization inhibitor such as, for example, butylated hydroxytoluene (BHT); hydroquinone; hydroquinone monomethyl ether; benzoquinone; chloranil; phenol; butyl hydroxyanaline (BHA); tertiary butyl hydroquinone (TBHQ); tocopherol (Vitamin E); and the like. Preferably, butylated hydroxytoluene (BHT) is used as the polymerization inhibitor. The polymerization inhibitors act as scavengers to trap free radicals in the composition and to extend the material's shelf life.

In one embodiment, a material referred to as "ALF" comprising camphorquinone (CQ); butylated hydroxytoluene (BHT); N,N-dimethylaminoneopentyl acrylate, gamma-methacryloxypropyl trimethoxy silane and methacrylic acid can be used in the composition.

### Fillers

Conventional filler materials such as inorganic fillers, which can be naturally-occurring or synthetic, can be added to the ink jet printable dental material and composition. Such materials include, but are not limited to, silica, titanium dioxide, iron oxides, silicon nitrides, glasses such as calcium, lead, lithium, cerium, tin, zirconium, strontium, barium, and aluminum-based glasses, borosilicate glasses, strontium borosilicate, barium silicate, lithium silicate, lithium alumina silicate, kaolin, quartz, and talc. Preferably, the silica is in the form of silanized fumed silica. Preferred glass fillers are silanized barium boron aluminosilicate and silanized fluoride barium boron aluminosilicate. Organic particles such as poly(methyl methacrylate), poly(methyl/ethyl methacrylate), crosslinked polyacrylates, polyurethanes, grounded polymerized polymerizable compounds of this invention, polyethylene, polypropylene, polycarbonates and polyepoxides , and the like also can be used as fillers.

The inorganic filler particles can be surface-treated with a silane compound or other coupling agent to improve bonding between the particles and resin matrix. Suitable silane compounds include, but are not limited to, gamma-methacryloxypropyltrimethoxysilane, gamma-mercaptopropyltriethoxysilane, gamma-aminopropyltrimethoxysilane, and combinations thereof.

### Pigments

Examples of the inorganic pigment include, but not limited to, black iron oxide, yellow iron oxide, ultramarine blue, brown iron oxide, titanium oxide, zinc flower, zinc oxide, iron oxide, aluminum oxide, silicone dioxide, talc, barium sulfate, calcium sulfate, red oxide, cobalt chrome green, Armenian blue, carbon black, mica, cobalt violet, molybdenum red, titanium cobalt green, molybdate orange, and the like. Examples of the organic pigments include Cromophtal Red-BRN 2-napthalenecarboxamide, azo pigments, polyazo pigments, azomethine pigments, isoindoline pigments, anthraquinone pigments, phthalocyanine pigments, benzimidazolone pigments, and the like.

Wax like polymerizable resins based pigmented ink like materials of this invention contains one or more pigments as coloring or shading agents. The pigments include inorganic pigments and organic pigments. The pigments may be modified to increase the dispersibility. For example, modified pigments having a silane group, a polymerizable silane group, dialkylaminomethyl group or dialkylaminoethylsulfonic acid group are preferred used. In an additional example, inorganic pigments can be surface-treated with a silane compound or other coupling agent to improve bonding between the particles and resin matrix and dispersion in materials. Suitable silane compounds include, but are not limited to, gamma-methacryloxypropyltrimethoxysilane, gamma-mercaptopropyltriethoxysilane, gamma-aminopropyltrimethoxysilane, and combinations thereof.

The term "pigment" refers to visible materials which are not soluble, but are suspended or dispersed as fine particles in the subject materials. The preferred solid pigments are those pigments with fine particles, such as Black Iron Oxide 7053, Yellow Iron Oxide 7055, Titanium Dioxide, Cromophtal Red-BRN 2-napthalenecarboxamide, N,N'-(2-chloro-1,4-phenylene) bis{4-{(2,5-dichlorophenyl) azo}-3-hydroxy-}, ultramarine blue and brown iron oxide 420. In addition, a fluorescing agent may be included, such as Lumilux Blue LZ fluorescing agent (dihydroxy terepthalate acid ester). The preferred wax-like polymerizable materials of this invention utilizes a pigment having particle sizes of less than one micron, which are easily jetted through a 20 to 50 micron ink jet nozzles. Although the pigment particles would tend to settle out of inks having liquid vehicles, the compatible nature of our wax-like polymerizable material with pigments prevents this potential separation during jetting at elevated temperature. The surface of pigments may be organically modified to improve its compatibility to resin matrix. Pigments may also be prepolymerized in resin matrix and then grounded to powder so as to enhance their suspension in low viscose molten resins. The wax-like polymerizable materials are desirable for ink jet 3D printers to print 3D dental devices in accordance with this invention, since they remain in a solid phase at room temperature during shipping, long term storage and the like. The dental devices produced from these polymerizable materials can be try-in and adjusted as needed. The wax-like polymerizable materials can be applied directly onto a support substrate to form the device. Since the wax-like materials solidify immediately upon contact with the substrate, migration of the material is prevented, and dimensional precision. The wax-like materials have improved properties over light-curable liquid materials.

Pigments can be added to the ink-jet printable materials of this invention. Pigments are desirable because they provide superior shade stability and stand up to UV light irradiation. The wax-like formulations of this invention overcome potential problems with pigment separation in several ways. First, the pigment particles are more effectively dispersed in the solution to prevent settling. Secondly, the pigment particles are milled so that they have smaller dimensions. Particularly, nano-sized fine inorganic and organic pigment particles can be dispersed in the formulations. Nano-sized organic pigments are the most preferred.

The ink jet printable wax-like polymerizable dental materials may include various inorganic and organic fillers, pigments, initiators, catalysts, stabilizers, plasticizers, fibers or their combinations. Preferred stabilizers are butylated hydroxytoluene (BHT) and the methyl ether of hydroquinone (MEHQ). The materials may also include compounds that impart radiopaque properties.

The wax-like polymerizable dental materials are able to rapidly solidify. Rapid solidification provides a combination of flowability and dimensional stability, depending on its temperature prior to polymerization. The materials are preferably able to rapidly partially recrystallize. Rapid recrystallizability provides densification of the polymeric products and a combination of flowability and dimensional stability, depending on temperature prior to polymerization. When polymerized, the crystallized phase melts effectively resulting in volume expansion, which offsets polymerization shrinkage. Thus, the polymeric products have low shrinkage and low stress restoration. "Crystallinity" as used herein refers to regularity and order within a material resulting in a heat of fusion of at least 1.0 J/g at and below 50°C. "Heat of Fusion" as used herein refers to enthalpy of fusion as determined by ASTM 793-95. Percent of crystallinity is determined by measuring the heat of fusion using differential scanning calorimetry according to ASTM E 793-95.

### Methods

### Ink-Jet Printing using Particulate Powder Layers in Powder Bed

In one ink-jet printing method, a printing head discharges a liquid "binder" material onto a layer of powder particulate. The binder fuses the powder particulate together to form the first cross-sectional layer of the object. Particulate powder, which is unbound, supports the object. A second layer of particulate powder is prepared and the liquid binder material is discharged into this layer to form the second cross-sectional layer. Once the object has been completely fabricated, it can be removed from the unbound powder and then cured to form a hardened object. The dental practitioner can place the object (for example, dental crown) in a patient's mouth as a "try-in" so that the comfort and fit of the crown can be checked. If this is satisfactory, the crown can be finished and polished using conventional techniques. In an alternative method, the dental crown, as it sits in an uncured condition, is placed inside of a patient's mouth. The margins, contacts, and occlusion of the crown can be checked. Although the wax-like polymerizable material of the crown is uncured at this point, it is dimensionally stable. The crown structure is retained as the material does not slump and substantially change its shape. Because the material is dimensionally stable, the crown structure can be adjusted to fit comfortably. Then, the crown structure can be cured so that it fully hardens, and it can be finished and polished as needed.

Multiple powder beds can be used in the method of this invention. This is advantageous, because the operator can change the powder layers during fabrication of the dental devices to achieve desirable aesthetics and physical and mechanical properties.

In this method, the above-described low viscosity, wax-like polymerizable material is used as the "liquid binder." The liquid binder can be clear or pigmented. Preferably, the liquid "binder" has a refractive index that closely matches the refractive index of the powder particulate. Clear and pigmented liquid binders can be applied to the powder particulate to form layers having different translucent properties. The resulting dental prostheses have excellent esthetics. The liquid binder material binds the powder particles and forms a solid, uncured dental prosthetic having good integrity and mechanical strength. The resulting dental prostheses are dimensionally stable. They maintain their shape and structure while resting in uncured condition. The prostheses can be subsequently cured by light irradiation.

In conventional 3D printing systems, the produced object is required to set, polymerize, crosslink, and dry prior to being removed from the unbound particulate composition. In the present invention, the liquid binder system comprising the wax-like polymerizable material allows the uncured dental object to be removed immediately from the unbound particulate composition. The resulting dental device has good mechanical integrity and any rough surfaces can be smoothed and worked thereon. Then, the dental device can be cured and finished.

The above-described wax-like polymerizable material can be dispensed from heated reservoirs, system and printing heads. The material is jetted onto the powder bed, solidifies, and binds the powder particles together. The wax-like polymerizable material can rapidly solidify and bind the powder particulate to form shape-stable dental devices. A layer of powder particulate is prepared in a powder bed. The particulate can be wetted and bonded in selected areas by jetting the heated wax-like polymerizable material onto the particulate. The selected areas correspond to the cross-sectional layers of the dental product that will be made as generated by a three-dimensional computer model. The polymerizable material acts as a binder. Successive layers of powder/wax-like polymerizable material are applied in this manner to build-up the object. After excess powder, which is not bound together, is removed, the final object can be cured. One advantage with using the wax-like polymerizable of this invention is its excellent dimensional stability. The material is.shape-stable due to its ability to rapidly solidify and crystallize. This means that excess liquid binder material can be used in the powder bed to minimize porosity in the final object. Heating and ultrasonic control systems can be used to reduce the viscosity of resin binder, better wet the powder bed, drive off air bubbles and reduce porosity. Moreover, if uncured resin powder particulate is used to form the layers, heat (for example, infrared (IR) or laser beams) can melt the powder particulate and binder resin together. This also will help reduce porosity in the finished object.

In addition, this invention also provides unique powder particulate systems. Resins of the wax-like polymerizable material, as described above, can be broken down and ground to form fine powder particles that can be used in the particulate powder bed. When the solid composite and resin particles made from the wax-like polymerizable material are uncured, they have unique properties. Particularly, they have good shape-stability at room temperature and meltable /flowability properties at elevated temperature. Furthermore, these composites and resin particles can be cured to form desired particle sizes. The properties of the wax-like material make the material particularly desirable for use as a particulate powder in the powder bed. However, other powder particle materials such as polymers, composites, pigments, and fillers such as silica, alumina, silicon nitride, and glasses can be used in the powder bed if desired. It is preferable that the uncured powder particles have a melting point higher than the uncured wax-like compounds (binder material) which is jetted from the ink-jet printer. In one embodiment, the ink-jet printing method using a particulate powder bed involves the steps of:
a) generating computer data corresponding to layers of the desired object;
b) providing a layer of powder particles;
c) providing several shades of wax-like polymerizable materials which are a fluid at a temperature between about 40°C to about 140°C;
d) providing clear wax-like polymerizable materials which are a fluid at temperature between about 40°C to about 140°C to build said object;
e) dispensing a layer of powder particles on a powder bed;
f) selectively dispensing the wax-like polymerizable materials at elevated temperature according to computer data to completely wet the specific powder particles and build a specific shaded layer of the object;
g) providing an environment that lowers the temperature of said dispensed material into a solid state and bonds said powder particles together;
h) repeating step e), f), and g) to form subsequent layers until said object is formed;
i) removing said object from unbound powder particles; and
j) curing said object (the object may be cured after it is tried in the patient's mouth and adjusted.)

### Ink-Jet Printing using Supporting System

In a second ink-jet printing method, the wax-like polymerizable material is not jetted into a particulate powder bed. Instead, a supporting material is used as a scaffold or foundation for building-up the three-dimensional object. An ink-jet printer is used to jet the wax-like polymerizable material and supporting material onto a working platform (support surface). The wax-like material changes from a shape-stable material to low viscosity flowable material when it is exposed to elevated temperatures. The support surface materials can be wax, water-soluble, or wax-like material or other materials as described further below.

The ink-jet nozzles impinge the melted materials onto the support surface. Upon contacting an atmospheric air environment, the discharged materials harden. The three-dimensional dental prosthesis and material supporting the prosthesis are built-up layer-by-layer using this method. Once the three-dimensional prosthesis has been completely fabricated, the supporting material is removed.

In general, there are three different approaches for fabricating the three-dimensional dental prosthesis using the ink-jet printing / supporting system method.

Following each of these approaches, the wax-like polymerizable material is heated to form a polymerizable liquid and the supporting material (or substrate) is also heated to form a flowable liquid. The ink-jet printer discharges droplets of the liquid phase wax-like polymerizable material and supporting material onto a solid platform in a pattern. Successive layers of the polymerizable material and supporting material are applied to form the denture or other dental device.

In a first approach, the wax-like polymerizable material is discharged onto a support surface to form a first cross-sectional layer of the dental prosthesis. Successive layers of the material are added until the denture, crown or other prosthesis is fabricated. Then, the prosthesis (for example, a denture) can be tried-in the patient's mouth. The patient bites down upon the denture so that the margins, contacts, and occlusion can be checked by the dentist. After completing the try-in step, the dentist makes any necessary adjustments, tries in the denture a second time, and finishes the denture so that it is ready for final curing. In the final curing step, the denture is exposed to light irradiation so that each layer is fully cured. The prosthesis, while in an uncured condition, is shape-stable. However, a dental practitioner can shape and mold the prosthesis slightly so that it fits over a targeted area inside of the mouth by applying sufficient pressure to the device. Then, the patient can bite down on the fitted prosthesis. As the patient bites down, the practitioner can check margins, contacts, and bite occlusion. The practitioner can handle and work the prosthesis in the mouth to adjust it and make it feel more comfortably. Because the prosthesis has some flexibility, the practitioner is able to remove the fitted device from the mouth rather easily. The prosthesis is not strongly adhered to the tooth structure or gum tissue at this time. However, the prosthesis has strong integrity and stability such that it is dimensionally stable. The shape of the prosthesis is maintained. There is no deformation of the prosthesis upon removing it from the mouth. Once the prosthesis has been removed, it can be cured and hardened by exposing it to light radiation using standard dental light-curing devices.

Different support surface materials can be used in the ink-jetting process including, for example, wax, starch, water-soluble solid materials such as polyethylene glycol, surfactants, water, glycols, flowable waxy substances, and mixtures thereof. Also, a dental casting slurry can be used as the supporting material. Once the three-dimensional object has been fabricated, the supporting materials can be removed. Various methods can be used to remove the supporting materials. For example, the supporting material can be removed by heating because of the difference in melting point or partially/fully polymerization states of the wax-like polymerizable material and supporting material. In another example, the supporting material can be removed by dissolving the material in water, because of the different water solubilities of the respective materials. In yet another example, the supporting material can be removed by mechanical/physical means because of the different mechanical strength and physical characteristics of the respective materials.

Optionally, a separating layer may be ink-jetted between the wax-like polymerizable material used to build the dental object and the supporting material. Using a separating layer helps make removing the printed dental device from the supporting surface easier. This separating layer acts as a releasing agent on the supporting surface. The separating layer can be made from materials such as water, fluorinated oils, glycols, surfactants, mineral oils, silicone oils, polymerizable materials, functional oils, waxes, flowable waxy substances, wax-like oils and combination thereof. For example, a dental casting, stone-like slurry can be used as the separating layer, and the fabricated prosthesis can be removed from the stone-like material. Subsequently, the stone-like material can be used to support the final cure of the prosthesis.

More particularly, to build the three-dimensional dental object, the above-described wax like polymerizable material is used. The material which is formulated with the required pigments may be cast into solid-colored sticks and placed in an ink jet printing device. Then the temperature is raised to a first elevated operating temperature so that a liquid phase wax-like polymerizable material with selective fluid properties is formed. The material is typically held as a liquid at this elevated temperature in the reservoir and print head of the ink jet printer.

Then, the liquid phase wax-like polymerizable material (and supporting material) can be directly applied in a predetermined pattern onto the platform in a layer-by-layer manner, with the print head moving in a two-dimensional direction. The liquid phase polymerizable material is printed "layer wise" onto the supporting material. The layered three-dimensional dental device may need to be cured in a light-curing unit to form a finished product. The resulting denture or other dental device should exhibit excellent shade and color properties.

As discussed above, a separating layer can be used in the ink-jet printing process. The separating layer is introduced between the supporting material and three-dimensional object-forming layer. Once the object has been fabricated, it can be removed easily from the separating layer. This ink-jet printing with separating layer method generally involves the steps of:
a) generating computer data corresponding to layers of said object;
b) providing several shades of wax-like polymerizable materials which are a fluid at temperature between 40°C to about 140°C;
c) providing a clear wax-like polymerizable materials which are a fluid at temperature between 40°C to about 140°C to build said object;
d) providing a supporting material;
e) providing a separating material which is a fluid at temperature between 40°C to about 140°C;
f) selectively dispensing the supporting material at elevated temperature according to computer data to form a supporting layer;
g) selectively dispensing the separating material at elevated temperature according to computer data to form a separating layer between building layer and supporting layer;
h) selectively dispensing the wax-like polymerizable materials at elevated temperature according to computer data to build a specific shaded layer of the object;
i) providing an environment that lowers the temperature of said dispensed build material and support material into a solid state;
j) repeating step f), g), h) and i) to form subsequent layers until said object is formed;
k) separating said object from supporting material; and
l) curing said object (the object may be cured after it is tried in the patient's mouth and adjusted.).

Different materials can be used to form a separating layer between the supporting material and wax-like, polymerizable object-building material. For example, the separating layer can be made from water, fluorinated oils, glycols, surfactants, mineral oils, silicone oils, polymerizable materials, functional oils, waxes, flowable waxy substances, wax-like oils and combination thereof. The separating material can be ink-jetted onto the supporting surface as described above. The separating layer provides a good release mechanism for removing the fabricated three-dimensional object from the supporting surface. However, it is not necessary that a separating layer be used in ever instance.

In other cases, the three-dimensional dental object can be fabricated and removed from the supporting surface without using a separating layer. For example, if a wax or water-soluble, wax-like material is used as the supporting material, it can be removed by melting or dissolving it, and a separating layer is not needed. This alternative method generally includes the following steps:
a) generating computer data corresponding to layers of said object;
b) providing several shades of wax-like polymerizable materials which are a fluid at temperature between 40°C to about 140°C;
c) providing a clear wax-like polymerizable materials which are a fluid at temperature between 40°C to about 140°C to build said object;
d) providing a supporting wax material;
e) selectively dispensing the supporting wax material at elevated temperature according to computer data to form a supporting layer;
f) selectively dispensing the wax-like polymerizable materials at elevated temperature according to computer data to build a specific shaded layer of the object;
g) providing an environment that lowers the temperature of said dispensed build material and support material into a solid state;
h) partially polymerizing the wax-like polymerizable materials by light irradiation;
i) repeating step e), f), g), and h) to form subsequent layers until said object is formed;
j) separating said object from supporting material; and
k) finally curing said object (the object may be cured after it is tried in the patient's mouth and adjusted).

In a second approach, an ink-jet printer discharges successive layers of wax-like, polymerizable material onto a platform to form the denture. Then, the shaped device is partially cured by exposing it to light irradiation. The device does not fully harden but maintains some flexibility. The partially cured denture can be placed in the mouth as a try-in. Once the denture has been properly fitted, it is fully cured by light irradiation.

Preferably, high-strength dental products are produced by the methods of this invention. In a preferred embodiment, the wax-like polymerizable material (with no reinforcing fillers) can be discharged from the ink-jet printer to produce the high-strength dental product. By the term, "high-strength" as used herein, it is meant that the products have a flexural modulus of at least 200,000 psi and a flexural strength of at least 5,000 psi. More preferably, the product has a flexural modulus of at least 300,000 psi and a flexural strength of at least 8,000 psi. Most preferably, the product has a flexural modulus of at least 350,000 psi and a flexural strength of at least 12,000 psi. "Flexural strength, and flexural modulus" as used herein refers to properties measured according to the methods of ASTM D790 (1997).

As described further in the Examples below, piezoelectric ink jet print heads can be used in the printing device to produce the three-dimensional object. Piezo print heads allow the use of pigmented materials and are able to handle materials with higher viscosity. Piezo print heads can vary the size of droplets so the printing speed and resolution can be adjusted. Both liquid and solid dental materials of this invention can be printed out by using two basic piezoelectric printing head technologies. More particularly, the printing heads used on the Phaser printers available from Xerox and LaserMaster DisplayMaker Express printers available from Spectra can be used to print the wax-like polymerizable dental material of this invention. The print heads used on the Xerox Phaser 8500 ink-jet printer are believed to be especially suitable.

Also, as described in the following examples, various formulations containing the wax-like polymerizable material can be prepared for use in an ink-jet printing device. It is important that the formulations have sufficiently low viscosity so that they can be handled and discharged easily from the ink-jet printing devices. At the same time, the formulations must be capable of producing dental products having sufficient mechanical strength and integrity. Several low viscosity wax-like polymerizable materials were prepared with black, yellow, blue, black and white pigments. The materials were successfully dispensed from a Xerox two-dimensional ink-jet printer (Phaser 8500), which uses a piezoelectric print head.

### EXAMPLES

### EXAMPLE 1

### Preparation of Oligomer

A reactor was charged with 1176 grams of trimethyl-1,6-diisocyanatohexane (5.59 mol) and 1064 grams of bisphenol A propoxylate (3.09 mol) under dry nitrogen flow and heated to about 65°C under positive nitrogen pressure. To this reaction mixture, 10 drops of catalyst dibutyltin dilaurate were added. The temperature of the reaction mixture was maintained between 65 °C and 140°C for about 70 minutes and followed by additional 10 drops of catalyst dibutyltin dilaurate. A viscous paste-like isocyanate end-capped intermediate product was formed and stirred for 100 minutes. To this intermediate product, 662 grams (5.09 mol) of 2-hydroxyethyl methacrylate and 7.0 grams of BHT as an inhibitor were added over a period of 70 minutes while the reaction temperature was maintained between 68°C and 90° C. After about five hours stirring under 70 °C, the heat was turned off, and oligomer was collected from the reactor as semi-translucent flexible solid and stored in a dry atmosphere.

### EXAMPLE 2

### Preparation of Monomer

A reaction flask was charged with 700 grams of 1,6-diisocyanatohexane and heated to about 70°C. under positive nitrogen pressure. To this reactor were added 1027 grams of 2-hydroxyethyl methacrylate, 0.75 gram of catalyst dibutyltin dilaurate and 4.5 grams of butylated hydroxy toluene (BHT). The addition was slow and under dry nitrogen flow over a period of two hours. The temperature of the reaction mixture was maintained between 70°C and 90°C for another two hours and followed by the addition of 8.5 grams of purified water. One hour later, the reaction product was discharged as clear liquid into plastic containers and cooled to form a white solid and stored in a dry atmosphere.

### EXAMPLE 3

### Preparation of Monomer

A reaction flask was charged with 168 grams of 1,6-diisocyanatohexane and heated to about 70°C under a positive nitrogen pressure. To this reactor were added 228 grams of 2-hydroxyethyl acrylate, 0.12 gram of catalyst dibutyltin dilaurate and 0.86 grams of butylated hydroxy toluene (BHT). The addition was slow and under dry nitrogen flow over a period of two hours. The temperature of the reaction mixture was maintained between 70°C and 85°C for another three hours and followed by the addition of 0.9 grams of purified water. One hour later, the reaction product was discharged as clear liquid into plastic containers and cooled to form a white solid and stored in a dry atmosphere.

### EXAMPLE 4

### Preparation of Monomer

A reaction flask was charged with 200 grams of octadecyl isocyanate and heated to about 78°C under a positive nitrogen pressure. To this reactor were added 90.6 grams of 2-hydroxyethyl methacrylate, 0.14 gram of catalyst dibutyltin dilaurate and 0.58 grams of butylated hydroxy toluene (BHT). The addition was slow and under dry nitrogen flow over a period of two hours. The temperature of the reaction mixture was maintained between 70°C and 85°C for another 3 hours, and the reaction product was discharged as clear liquid into plastic containers and cooled to form a white solid and stored in a dry atmosphere.

### Particulate Material

The particulate composition includes up to 100 wt% of glass, ceramic or bio-ceramic powders or various ground polymeric, uncured and cured, composite particles. One specific example of particulate material which can be prepared is a blend of silanated fumed silica and silanated barium aluminoflurosilicate glasses with various particle sizes.

### EXAMPLE 5

### Particulate Material

A mixture of silanated fumed silica (SiO₂) having an average particles size of from about 0.01 to about 0.04 micrometers, ultrafine silanated barium aluminoflurosilicate glass particles BAFG having an average particle size of from about 0.1 to about 1 micrometer and silanated barium aluminoflurosilicate glass particles BAFG having an average particle size of from about 1 to about 10 micrometers at a weight ratio of 20:60:20 was prepared.

### EXAMPLE 6

### Particulate Material

A mixture of silanated fumed silica (SiO₂) having an average particles size of from about 0.01 to about 0.04 micrometers, ultrafine silanated barium aluminoflurosilicate glass particles BAFG having an average particle size of from about 0.1 to about 1 micrometer at a weight ratio of 5:95 was prepared.

### EXAMPLE 7

### Particulate Material

A mixture of ultrafine silanated barium aluminoflurosilicate glass particles BAFG having an average particle size of from about 0.1 to about 1 micrometer and silanated barium aluminoflurosilicate glass particles BAFG having an average particle size of from about 1 to about 10 micrometers at a weight ratio of 50:50 was prepared.

### EXAMPLE 8

### Particulate Material

An ultrafine silanated barium aluminoflurosilicate glass particles BAFG having an average particle size of from about 0.1 to about 1 micrometer was prepared.

### EXAMPLE 9

### Particulate Material made from Wax-Like Polymerizable Material

A wax-like polymerizable dental material was prepared by stirring at 85°C a liquid mixture of 38.65 grams of oligomer made following the procedure of Example 1; 46.5 grams of the compound of Example 2; 6.5 grams of the compound of Example 3; 8.0 grams of the compound of Example 4; and 0.35 grams of 2,4,6-trimethylbenzoyldiphenylphosphine oxide, (Lucirin TPO made by BASF). This wax-like material was light cured and subsequently ground to form particulate powder containing particles having an average particle size in the range of about 10 to about 150 micrometers.

### EXAMPLE 10

### Particulate Material made from

### Wax-Like Polymerizable Composite Material

A wax-like polymerizable dental composite material was prepared by stirring at 85°C a liquid mixture of 4.12 grams of oligomer made following the procedure of Example 1; 4.20 grams of the compound of Example 2; 1.45 grams of the compound of Example 3; 5.45 grams of 7,7,9-trimethyl-4,13-dioxo-3,14 dioxa-5,12-diazahexadecane-1,16-diol dimethacrylate; 6.00 grams of Ethoxylated bisphenol A dimethacrylate (SR348 from Sartomer Company, Inc.); 2.00 grams of silanated fumed silica (SiO₂) having an average particle size of from about 0.01 to about 0.04 micrometers; 62 grams of silanated barium aluminoflurosilicate glass particles BAFG having an average particle size of from about 0.1 to about 1 micrometer; 14 grams of silanated barium aluminoflurosilicate glass particles BAFG having an average particle size of from about 1 to about 10 micrometers; and 0.28 grams of visible light initiating solution containing 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane. This wax-like material was light cured and subsequently ground to form particulate powder containing particles having an average particle size in the range of about 10 to about 150 micrometers.

### EXAMPLE 11

### Polymerizable Particulate Material made from

### Wax-Like Polymerizable Material

A wax-like polymerizable dental material was prepared by stirring at 85°C a liquid mixture of 40 grams of oligomer made following the procedure of Example 1; 39.25 grams of compound of Example 2; 20 grams of compound of Example 3; 0.75 grams of visible light initiating solution containing 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane. This wax-like material was subsequently cryogenic ground to form particulate powder containing particles having an average particle size in the range of about 10 to about 150 micrometers.

### EXAMPLE 12

### Polymerizable Particulate Material made from

### Wax-Like Polymerizable Material

A wax-like polymerizable dental composite material was prepared by mixing a mixture of 51 grams of oligomer made following the procedure of Example 1; 28 grams of compound of Example 2; 18 grams of compound of Example 3; 59.93 grams of silanated fumed silica (SiO₂) having an average particles size of from about 0.01 to about 0.04 micrometers; 179.8 grams of silanated barium aluminoflurosilicate glass particles BAFG having an average particle size of from about 0.1 to about 1 micrometer; 59.93 grams of silanated barium aluminoflurosilicate glass particles BAFG having an average particle size of from about 1 to about 10 micrometers, 0.08 grams of #115 Phosphor; 0.0192 grams of Lumilux Blue LZ fluorescing agent (dihydroxy terepthalate acid ester); 0.4 grams of Lucirin-TPO (2,4,6-Trimethylbenzoyldiphenylphosphine oxide); and 2.0 grams of visible light initiating solution containing 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane. This wax-like composite material was subsequently cryogenic ground to form powders with average particle sizes ranging from about 10 to about 150 micrometers.

### Crystallizable Liquid Phase Binder

A rapidly crystallizable liquid phase binder is used to bind the powder particulate in a powder bed to fabricate the dental object. The liquid phase binder is discharged into the powder bed from an ink-jet printer. The liquid phase may contain acrylate or methacrylate monomers or oligomers and light curable initiators. Preferably, this liquid phase melts at elevated temperatures and solidifies at room temperature rapidly to bind (or wet) the powder phase. This results in shape-stable three-dimensional objects being formed immediately. The objects have sufficient mechanical integrity and strength due to rapid crystallization of the liquid phase binder. It is important that the intermediate uncured object have sufficient integrity and mechanical strength in order to produce a finally cured object that will not distort.

### EXAMPLE 13

### Dental Materials

A polymerizable dental material was prepared by stirring at 85°C a liquid mixture of 6.42 grams of oligomer made following the procedure of Example 1; 16.64 grams of the compound of Example 2; 7.05 grams of the compound of Example 3; 26.39 grams of 1,14-tetradecanedimethacrylate, and 0.21 grams of 2,4,6-trimethylbenzoyldiphenylphosphine oxide (Lucirin TPO available from BASF).

### EXAMPLE 14

### Dental Materials

A wax-like polymerizable dental material was prepared by stirring at 85°C a liquid mixture of 38.65 grams of oligomer made following the procedure of Example 1; 46.5 grams of the compound of Example 2; 6.5 grams of the compound of Example 3, and 8.0 grams of the compound of Example 4; and 0.35 grams of 2,4,6-trimethylbenzoyldiphenylphosphine oxide, (Lucirin TPO available from BASF).

### EXAMPLE 15

### Dental Materials

A polymerizable dental material was prepared by stirring at 85°C a liquid mixture of 5 grams of oligomer made following the procedure of Example 1; 15 grams of the compound of Example 2; 5 grams of the compound of Example 3; 5.0 grams of 1,6-hexanediol dimethacrylate; 30 grams of 1,14-tetradecanedimethacrylate; 1.0 grams of visible light initiating solution containing 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane.

### EXAMPLE 16

### Dental Materials

A polymerizable dental material was prepared by stirring at 85°C a liquid mixture of 15 grams of the compound of Example 2; 5 grams of the compound of Example 3; 20 grams of 1,14-tetradecanedimethacrylate; 15 grams of dimethylol tricyclodecane diacrylate; 5 grams of 7,7,9-trimethyl-4,13-dioxo-3,14 dioxa-5,12-diazahexadecane-1,16-diol dimethacrylate; 10 grams of Genomer 4256 (aliphatic polyester urethane methacrylate supplied by Rohm America Inc.); 0.2 grams of 2,4,6-trimethylbenzoyldiphenylphosphine oxide (Lucirin TPO supplied by BASF); and 0.25 grams of 2,4,6- trimethylbenzoyldiphenylphosphine oxide (Lucirin TPO supplied by BASF).

### EXAMPLE 17

### Dental Materials

A polymerizable dental material was prepared by stirring at 85°C a liquid mixture of 36.15 grams of the compound of Example 1; 29.2 grams of the compound of Example 2; 14.5 grams of the compound of Example 3; 19.8 grams of cyclohexane dimethanol dimetharylate; and 0.35 grams of 2,4,6- trimethylbenzoyldiphenylphosphine oxide (Lucirin TPO supplied by BASF).

### EXAMPLE 18

### Dental Materials

A polymerizable dental material was prepared by stirring at 85°C a liquid mixture of 77.5 grams of oligomer made under the procedure of Example 1; 91.0 grams of the compound of Example 2; 13.0 grams of the compound of Example 3; 17.0 grams of the compound of Example 4; and 0.7 grams of 2, 4, 6- trimethylbenzoyldiphenylphosphine oxide, (Lucirin TPO supplied by BASF); 0.2 grams of red acetate fibers; and 0.6 grams of pigments.

### EXAMPLE 19

### Dental Materials

A light curable wax-like polymerizable dental material was prepared by stirring at 85°C a liquid mixture of 48.22 grams of oligomer made following the procedure of Example 1; 28.5 grams of the compound of Example 2; 9.0 grams of the compound of Example 3; 7.5 grams of the compound of Example 4; 2.5 grams of stearyl acrylate; 3.5 grams of bisphenol A dimethacrylate; 0.35 grams of 2,4,6-trimethylbenzoyldiphenylphosphine oxide (Lucirin TPO supplied by BASF); 0.2 grams of visible light initiating solution containing 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane, and 0.23 gram of pigments.

### EXAMPLE 20

### Dental Materials

A light curable wax-like polymerizable dental material was prepared by stirring at 85°C a liquid mixture of 49.5 grams of oligomer made following the procedure of Example 1; 40 grams of compound of Example 2; 9.75 grams of the compound of Example 3; and 0.75 grams of visible light initiating solution containing 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane.

### EXAMPLE 21

### Dental Materials

A polymerizable dental material was prepared by stirring at 85°C a liquid mixture of 20 grams of oligomer made following the procedure of Example 1, 24 grams of the compound of Example 2; 12 grams of the compound of Example 3; 6 grams of 7,7,9-trimethyl-4,13-dioxo-5,12-diazahexadecane-1,16-diol dimethacrylate, 40 grams of 1,14-tetradecanedimethacrylate, and 1.0 grams of visible light initiating solution containing 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane.

### EXAMPLE 22

### Fabrication of Dental Product

The material of Example 20 and one of its corresponding pigmented versions are heated in several 85°C reservoirs of an ink-jet printer and applied to a work surface in a micro drop-wise manner as controlled by a computer. This process can be used to form a dental crown in a layer- by-layer manner. The external surfaces of the crown structure are slightly smoothed with a gentle blow of hot air. After a sealer is applied, the crown structure is cured in a light-curing unit. This process produces a provisional crown that a dentist can subsequently try-in a patient's mouth to check for comfort and fit. Once any adjustements are made, the crown is relined or cemented on the crown-prepped tooth.

### EXAMPLE 23

### Fabrication of Dental Product

The materials of Example 18 and 19 and/or at least three corresponding pigmented versions are heated and melted on demand in at least four reservoirs (about 120° to 130°C) of an ink-jet printer and then mixed at the print head. The melted material is applied in a micro drop-wise manner to a pre-formed baseplate as controlled by a computer. In this manner, a gum-like pink denture base and lightly colored teeth are fabricated on top of a pre-formed baseplate. The surfaces of the denture are slightly smoothed with a gentle blow of hot air. The dentist can try-in the denture in a patient's mouth to check for comfort and fit. After try-in, a supporting model may be built on the tissue side of the uncured denture by pouring in gypsum or injecting die silicone prior to curing for optimized dimensional stability. Then, an ABC (air barrier coating) or sealer is applied and the object is cured in a light-curing unit to form a final denture. After the denture is finished and polished, it is delivered to the patient.

### EXAMPLE 24

### Fabrication of Dental Product

The material of Example 18 is heated in a 110°C reservoir of an ink-jet printer and applied in a micro drop-wise manner to a working surface. The melted resin immediately crystallizes and forms a solid layer. In this manner, a crown can be fabricated layer-by- layer. The external surfaces of the crown are slightly smoothed with a gentle blow of hot air. Optionally, a die silicone supporting post may be built in the cavity of the uncured crown prior to curing. This post optimizes the dimensional stability of the crown structure. After sealer is applied, the crown is cured in a light-curing unit. A dentist can subsequently try-in the crown in a patient's mouth to check for comfort and fit. Once any adjustments are made, the crown can be relined or cemented on the crown-prepped tooth. curing step. The die silicone helped optimize the dimensional stability of the crown. Then, a sealer was applied and the crown was cured in a light unit. After the provisional crown was finished and polished, it was ready for subsequent try-in, cement or reline at a dentist's office.

### EXAMPLE 25

### Fabrication of Dental Product

The material of Example 21 and their corresponding two lightly pigmented versions are heated in three 85°C reservoirs of an ink-jet printer. The melted material is applied in a micro drop-wise manner to a working surface. The material is applied in a layer-by-layer manner to build the dental object. This process can be used to make a dental bridge having three layers of different shades. Optionally, die silicone supporting posts may be built on the uncured bridge prior to the curing step. This step improves the dimensional stability of the bridge. Then, the surfaces of the bridge can be slightly smoothed with a gentle blow of hot air. After a sealer is applied, the bridge structure can be cured in a light-curing unit.

### EXAMPLE 26

### Fabrication of Dental Product

The particulate composition of Example 5 is spread out as a first particulate layer in a particulate build bin. The heated liquid binder layer of Example 15 is then applied to the first particulate layer from a heated ink-jet printing head. A first uncured layer of a crown structure having a specific shape is formed in this manner. Then a second particulate layer is applied to the first uncured layer of the crown structure. From the heated ink-jet printing head, additional liquid binder of Example 15 is applied to the second particulate layer. This forms a second uncured layer of the crown structure. This procedure is continued until the desired three-dimensional (3D) crown structure is formed. Next, the crown object is removed from the unbound particulate composition and the surface of the crown is slightly smoothed with a gentle blow of hot air. After sealer is applied, the crown structure is cured in a Triad 2000 light-curing unit for 10 minutes. This curing step produces a final crown product, which can be relined or cemented on a crown-prepped tooth in a patient's mouth.

### EXAMPLE 27

### Fabrication of Dental Product

The particulate composition of Example 12 is spread out as a first particulate layer in a container. The liquid binder of Example 15 is then applied to the first particulate layer from a heated tip of the ink-jet printing head. A first uncured layer of a crown structure having a specific shape is formed in this way. Then, a second particulate layer is applied to the first uncured layer of crown. From the heated ink-jet printing head, additional liquid binder of Example 15 is applied to the second particulate layer. This forms a second uncured layer of the crown structure. This procedure is continued until the desired three-dimensional (3D) crown structure is formed. Next, the crown object is removed from the unbound particulate composition and the surface of the crown is slightly smoothed with a gentle blow of hot air. Optionally, a die silicone supporting post may be built in the cavity of the uncured crown prior to curing. This step improves the dimensional stability of the crown structure. After sealer or ABC is applied, the crown structure can be cured in a Triad 2000 light-curing unit for 10 minutes. This curing step produces a final crown product,

### EXAMPLE 28

### Fabrication of Dental Product

The particulate composition of Example 9 is spread out as a first particulate layer in a container. A liquid binder selected from Example 17, 18 and their corresponding three pigmented versions is then applied to the first particulate layer from a heated tip in the ink-jet printing head. A first uncured layer of the denture product having a specific shape is formed in this way. Next, a second particulate layer is applied to the first uncured layer of the denture. Additional liquid binder layer selected from Example 17, 18 and their corresponding three pigmented versions is applied to the second particulate layer from a heated tip. This forms the second uncured layer of the denture. This layer- by-layer fabrication method continues until a desired three-dimensional denture object is formed. This denture object is removed from the unbound particulate composition and the surface of denture is slightly smoothed with a gentle blow of hot air. The denture object, in its uncured condition, can be sent to a dentist's office for try-in. After try-in and modification as needed, ABC or sealer can be applied on the surface of the denture. A supporting model may be built on the tissue side of this uncured denture by pouring in gypsum or injecting die silicone prior to cure for optimized dimensional stability. The denture object can be cured in light processing unit for 10 minutes to provide a final denture.

### EXAMPLE 29

### Preparation of Monomer

A reaction flask was charged with 6.80 grams of 1, 10-decanediol (0.039 mol) and 12.15 grams (0.078 mol) of 2-isocyanatoethyl methacrylate under dry nitrogen flow and heated to about 60°C under positive nitrogen pressure. To this reaction mixture, 2 drops of catalyst dibutyltin dilaurate were added. The temperature of the reaction mixture was maintained between 60°C and 67°C and clear viscous liquid was formed. Five hours later, the heat was turned off, the flask was removed, and monomer was collected as viscous liquid and cooled to form white solid that was stored in a dry atmosphere.

### EXAMPLE 30

### Preparation of Monomer

A reaction flask was charged with 6.22 grams of 2,5-dimethyl-2,5-hexanediol (0.0425 mol) under dry nitrogen flow and heated to about 59°C under positive nitrogen pressure. Comonomer 2-isocyanatoethyl methacrylate (13.2 grams, 0.0851 mol) was charged into this reactor under constant stirring and followed by the addition of three drops of catalyst dibutyltin dilaurate. The temperature of the reaction mixture was maintained between 59°C and 63°C and a white solid was formed in half an hour. Seven hours later, the heat was turned off, the flask was removed and monomer was collected as white solid and stored in a dry atmosphere.

### EXAMPLE 31

### Preparation of Monomer

A reaction flask was charged with 43.8 grams of 1, 12-diisocyanatododecane and heated to about 85°C under a positive dry air pressure. To this reactor was added 0.06 gram of catalyst dibutyltin dilaurate. 45.0 grams of 2-hydroxyethyl methacrylate, and 0.07 grams of butylated hydroxy toluene (BHT) was then slowly added under dry air flow over a period of 41 minutes. The temperature of the reaction mixture was maintained between 80°C and 90°C for another 1.3 hours and followed by the addition of 0.12 grams of purified water. 50 minutes later, the reaction product was discharged as clear liquid into plastic containers and cooled to form a white solid that was stored in a dry atmosphere.

### EXAMPLE 32

### Preparation of Monomer

A reaction flask was charged with 40 grams of 1, 12-diisocyanatododecane and heated to about 85°C under a positive dry air pressure. To this reactor was added 0.06 gram of catalyst dibutyltin dilaurate. 36.5 grams of 2-hydroxyethyl acrylate, and 0.07 grams of butylated hydroxy toluene (BHT) was then slowly added under dry air flow over a period of 41 minutes. The temperature of the reaction mixture was maintained between 80°C and 90°C for another 2 hours and followed by the addition of 0.10 grams of purified water. One hour later, the reaction product was discharged as clear liquid into plastic containers and cooled to form a white solid that was stored in a dry atmosphere.

### EXAMPLE 32B

### Preparation of Monomer

A reaction flask was charged with 5.50 grams of 1, 12-diisocyanatododecane and heated to about 73°C under a positive dry air pressure. To this reactor was added 0.02 gram of catalyst dibutyltin dilaurate. 5.75 grams of hydroxypropyl methacrylate, and 0.03 grams of butylated hydroxy toluene (BHT) were then slowly added under dry air flow over a period of 9 minutes. The temperature of the reaction mixture was maintained between 74°C and 86°C for another 2.5 hours and the reaction product was discharged as clear liquid into plastic containers and cooled to form a semi-opaque white solid and stored in a dry atmosphere.

### EXAMPLE 32C

### Preparation of Monomer

A reaction flask was charged with 9.95 grams of 1, 12-diisocyanatododecane and heated to about 60°C under a positive dry air pressure. To this reactor was added 0.04 gram of catalyst dibutyltin dilaurate. 12.58 grams of hydroxybutyl methacrylate, and 0.03 grams of butylated hydroxy toluene (BHT) were then slowly added under dry air flow over a period of 52 minutes. The temperature of the reaction mixture was maintained between 80°C and 89°C for another 2.5 hours and the reaction product was discharged as clear liquid into plastic containers and cooled to form a semi-opaque solid and stored in a dry atmosphere.

### EXAMPLE 32D

### Preparation of Monomer

A reaction flask was charged with 10.85 grams of 1, 12-diisocyanatododecane and heated to about 85°C under a positive dry air pressure. To this reactor was added 0.04 gram of catalyst dibutyltin dilaurate. 29.0 grams of 2-hydroxypropyl 2-(methacryloyloxy)-ethyl phthalate, and 0.11 grams of butylated hydroxy toluene (BHT) were then slowly added under dry air flow over a period of 54 minutes. The temperature of the reaction mixture was maintained between 85°C and 95°C for another 2.5 hours and the reaction product was discharged as clear viscous liquid into plastic containers and cooled to form a gel-like solid and stored in a dry atmosphere.

### EXAMPLE 33

### Dental Materials

A polymerizable dental material was prepared by stirring at 95°C a liquid mixture of 16.7 grams of oligomer made the procedure of Example 1, 0.05 gram of Cromophtal Red-BRN {2-napthalenecarboxamide, N,N'-(2-chloro-1,4-phenylene) bis(4-[(2,5-dichlorophenyl) azo)-3-hydroxy-]}, 56.1 grams of compound of Example 2, 56.1 grams of compound of Example 3, 2.0 grams of butylated hydroxy toluene (BHT) and 1.0 gram of 2,4,6- trimethylbenzoyldiphenylphosphine oxide (Lucirin TPO made by BASF). This polymerizable dental material solidified to form light red wax-like solid. 25 grams of this material was formed into a solid block in the form of Xerox® Solid Ink 8500/8550 Magenta. This block has a melting viscosity of 12 cps at 130°C, 21 cps at 120°C, 27.5 cps at 110°C and 82 cps at 85°C.

### EXAMPLE 34

### Dental Materials

A polymerizable dental material was prepared by stirring at 95°C a liquid mixture of 6 grams of oligomer made following the procedure of Example 1; 0.02 gram of Cromophtal Red-BRN (2-napthalenecarboxamide, N,N'-(2-chloro-1,4-phenylene) bis(4-[(2,5-dichlorophenyl) azo)-3-hydroxy-]}; 20 grams of the compound of Example 31; 20 grams of the compound of Example 32, 0.2 grams of butylated hydroxy toluene (BHT) and 0.2 grams of 2,4,6- trimethylbenzoyldiphenylphosphine oxide (Lucirin TPO made by BASF). This polymerizable dental material solidified to form a light red wax-like solid.

### EXAMPLE 35

### Dental Materials

A polymerizable dental material was prepared by stirring at 95°C a liquid mixture of 0.02 gram of Cromophtal Red-BRN {2-napthalenecarboxamide, N,N'-(2-chloro-1,4-phenylene) bis(4-[(2,5-dichlorophenyl) azo)-3-hydroxy-]}; 20 grams of the compound of Example 31; 20 grams of the compound of Example 32; 0.2 grams of butylated hydroxy toluene (BHT); and 0.4 grams of visible light initiating solution containing 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane. This polymerizable dental material solidified to form a light red wax-like solid.

### EXAMPLE 36

### Dental Materials

A block of polymerizable dental material made in Example 33 was used in a Xerox@ Phaser 8500 ink-jet printer, and the material was successfully printed out on paper (two-dimensional).

### EXAMPLE 37

### Dental Materials

A polymerizable dental material was prepared by stirring at 95°C a liquid mixture of 2.00 grams of oligomer made following the procedure of Example 1; 0.0002 gram of Cromophtal Red-BRN {2-napthalenecarboxamide, N,N'-(2-chloro-1,4-phenylene) bis(4-[(2,5-dichlorophenyl) azo)-3-hydroxy-]}; 0.001 gram of yellow iron oxide 7055; 8.00 grams of the compound of Example 31; 8.00 grams of the compound of Example 32C, 1.80 grams of the compound of Example 32D, 0.2 grams of butylated hydroxy toluene (BHT); 0.5 gram of 2,4,6- trimethylbenzoyldiphenylphosphine oxide (Lucirin TPO made by BASF); and 0.5 gram of visible light initiating solution containing 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane. This polymerizable dental material solidified to form pink wax-like solid and was formed into a solid block of material similar to the form of Xerox® Solid Ink 8500/8550 Black. This block has a viscosity of 13 cps at 130°C.

### EXAMPLE 38

### Printing of Dental Materials

A block of polymerizable dental material made in Example 37 was used in a Xerox® Phaser 8500 ink-jet printer, and the material was successfully printed out on paper (two-dimensional).

## Claims

1. A method for making a three-dimensional dental prosthesis containing multiple cross-sectional layers, comprising the steps of:
a. applying wax-like polymerizable material onto a support surface to form a multi-layered, uncured prosthesis, each layer of the prosthesis being formed by an ink-jet printer jetting the polymerizable material onto the support surface;
b. placing the prosthesis in the mouth of a patient and shaping the prosthesis over a targeted area by applying sufficient pressure;
c. removing the shaped prosthesis from the mouth;
d. irradiating the prosthesis with curing light so that the prosthesis fully cures and hardens.

2. The method of claim 1, wherein the polymerizable material comprises a polymerizable acrylic compound and polymerization initiation system capable of being activated by light.

3. The method of claim 2, wherein the polymerizable material comprises a mixture of polymerizable acrylic oligomers and monomers.

4. The method of claim 2, wherein the polymerizable material further comprises particulate filler, wherein the particulate filler is preferably selected from the group consisting of silica, alumina, silicon nitride, and glass compounds.

5. The method of claim 2, wherein the polymerization initiation system comprises a photoactive agent selected from the group consisting of camphorquinone; 2,4,6 trimethylbenzoyldiphenyl phosphine oxide; and ethyl (4-N,N-dimethylamino) benzoate.

6. The method of claim 2, wherein the polymerizable material further comprises pigment, wherein the pigment is preferably selected from organic and inorganic pigments, or wherein the pigment is preferably selected from the group consisting of black iron oxide 7053, yellow iron oxide 7055, titanium dioxide, cromophtal red-BRN 2-napthalenecarboxamide, N,N'-(2-chloro-l,4-phenylene) bis{4-{(2,5- dichlorophenyl) azo }-3-hydroxy-}, ultramarine blue, brown iron oxide 420, and mixtures thereof.

7. The method of claim 1, wherein the prosthesis is irradiated with blue visible light having a wavelength in the range of about 400 to about 500 nm, or wherein the patient bites down prior to removing the uncured prosthesis from the mouth so that the fit of the prosthesis can be checked, or wherein a separating material is jetted from the ink-jet printer and deposited between the supporting material and wax-like polymerizable material.

8. A method for making a three-dimensional dental prosthesis containing multiple cross-sectional layers, comprising the steps of:
a. applying wax-like polymerizable material into a powder bed comprising particulate powder material to form a multi-layered, uncured prosthesis; each layer of the prosthesis being formed by an ink-jet printer jetting the polymerizable material into a layer of the particulate powder so that the particulate bonds in selected areas;
b. placing the prosthesis in the mouth of a patient and shaping the prosthesis over a targeted area by applying sufficient pressure;
c. removing the shaped prosthesis from the mouth;
d. irradiating the prosthesis with curing light so that the prosthesis fully cures and hardens.

9. The method of claim 8, wherein the polymerizable material comprises a polymerizable acrylic compound and polymerization initiation system capable of being activated by light.

10. The method of claim 9, wherein the polymerizable material comprises a mixture of polymerizable acrylic oligomers and monomers.

11. The method of claim 8, wherein the powder bed material comprises particulate powder having a melting point higher than the jetted wax-like polymerizable material.

12. The method of claim according to claim 11, wherein the particulate powder further comprises pigment.

13. The method of claim 12, wherein the pigment is selected from organic and inorganic pigments.

14. The method of claim 8, wherein the wax-like polymerizable material further comprises pigment, wherein the pigment is selected from organic and inorganic pigments.

## Patentansprüche

1. Verfahren zur Herstellung einer dreidimensionalen Zahnprothese, die mehrere Querschnittschichten enthält, umfassend die folgenden Schritte:
a. Aufbringen eines wachsähnlichen polymerisierbaren Materials auf die Oberfläche eines Trägers, um eine mehrschichtige, ungehärtete Prothese zu bilden, wobei jede Schicht der Prothese durch einen Tintenstrahldrucker gebildet wird, der das polymerisierbare Material auf die Oberfläche des Trägers ausstößt,
b. Platzieren der Prothese im Mund eines Patienten und Formen der Prothese über einem Zielbereich durch Anlegen eines ausreichenden Drucks;
c. Entfernen der geformten Prothese aus dem Mund;
d. Bestrahlen der Prothese mit härtendem Licht, so dass sich die Prothese gänzlich verfestigt und härtet.

2. Verfahren nach Anspruch 1, wobei das polymerisierbare Material eine polymerisierbare Acrylverbindung und eine Polymerisationsinitiator-System umfasst, das durch Licht aktiviert werden kann.

3. Verfahren nach Anspruch 2, wobei das polymerisierbare Material eine Mischung aus polymerisierbaren Acryl-Oligomeren und Acryl-Monomeren umfasst.

4. Verfahren nach Anspruch 2, wobei das polymerisierbare Material ferner einen partikulären Füllstoff umfasst, wobei der partikuläre Füllstoff bevorzugt ausgewählt ist aus der Gruppe, bestehend aus Siliciumdioxid, Aluminiumoxid, Siliciumnitrid und Glasverbindungen.

5. Verfahren nach Anspruch 2, wobei das Polymerisationsinitiator-System ein photoaktives Mittel umfasst, ausgewählt aus der Gruppe, bestehend aus Campherchinon; 2,4,6-Trimethylbenzoyldiphenyl-Phosphinoxid; und Ethyl(4-N,N-dimethylamino)benzoat.

6. Verfahren nach Anspruch 2, wobei das polymerisierbare Material ferner ein Pigment umfasst, wobei das Pigment bevorzugt ausgewählt ist aus organischen und anorganischen Pigmenten oder wobei das Pigment bevorzugt ausgewählt ist aus der Gruppe, bestehend aus Eisenoxidschwarz 7053, Eisenoxidgelb 7055, Titandioxid, Cromophtal Red-BRN-2-Naphtalencarboxamid, N,N'-(2-Chlor-1,4-phenylen)bis{4-{(2,5-dichlorphenyl)azo}-3-hydroxy-}, Ultramarinblau, Eisenoxidbraun 420 und Mischungen derselben.

7. Verfahren nach Anspruch 1, wobei die Prothese mit blauem sichtbaren Licht mit einer Wellenlänge in einem Bereich von ungefähr 400 bis ungefähr 500 nm bestrahlt wird oder wobei der Patient eine Bissprobe durchführt, bevor die ungehärtete Prothese aus dem Mund entfernt wird, so dass die Passform der Prothese überprüft werden kann, oder wobei ein Trennmaterial aus dem Tintenstrahldrucker ausgestoßen wird und zwischen dem Trägermaterial und dem wachsähnlichen polymerisierbaren Material eingelegt wird.

8. Verfahren zur Herstellung einer dreidimensionalen Zahnprothese, die mehrere Querschnittschichten enthält, umfassend die folgenden Schritte:
a. Aufbringen eines wachsähnlichen polymerisierbaren Materials in ein Pulverbett, das ein partikuläres Pulvermaterial umfasst, um eine mehrschichtige, ungehärtete Prothese zu bilden, wobei jede Schicht der Prothese durch einen Tintenstrahldrucker gebildet wird, der das polymerisierbare Material in die Schicht des partikulären Pulvers ausstößt, so dass das Partikulat in ausgewählten Bereichen bindet;
b. Platzieren der Prothese im Mund eines Patienten und Formen der Prothese über einem Zielbereich durch Anlegen eines ausreichenden Drucks;
c. Entfernen der geformten Prothese aus dem Mund;
d. Bestrahlen der Prothese mit härtendem Licht, so dass sich die Prothese gänzlich verfestigt und härtet.

9. Verfahren nach Anspruch 8, wobei das polymerisierbare Material eine polymerisierbare Acrylverbindung und eine Polymerisationsinitiator-System umfasst, das durch Licht aktiviert werden kann.

10. Verfahren nach Anspruch 9, wobei das polymerisierbare Material eine Mischung aus polymerisierbaren Acryl-Oligomeren und Acryl-Monomeren umfasst.

11. Verfahren nach Anspruch 8, wobei das Material des Pulverbetts ein partikuläres Pulver mit einem Schmelzpunkt umfasst, der höher ist als der des ausgestoßenen wachsähnlichen polymerisierbaren Materials.

12. Verfahren nach Anspruch 11, wobei das partikuläre Pulver ferner ein Pigment umfasst.

13. Verfahren nach Anspruch 12, wobei das Pigment ausgewählt ist aus organischen und anorganischen Pigmenten.

14. Verfahren nach Anspruch 8, wobei das wachsähnliche polymerisierbare Material ferner ein Pigment umfasst, wobei das Pigment ausgewählt ist aus organischen und anorganischen Pigmenten.

## Revendications

1. Procédé de fabrication d'une prothèse dentaire tridimensionnelle contenant de multiples couches transversales, comprenant les étapes suivantes :
a. application d'une matière polymérisable de type cire sur une surface de support pour former une prothèse multicouche, non durcie, chaque couche de la prothèse étant formée par projection par une imprimante à jet d'encre de la matière polymérisable sur la surface de support ;
b. positionnement de la prothèse dans la cavité buccale d'un patient et façonnage de la prothèse sur une zone ciblée par application d'une pression suffisante ;
c. retrait de la prothèse façonnée de la cavité buccale ;
d. exposition de la prothèse à une lumière de durcissement de façon que la prothèse se solidifie complètement et durcisse.

2. Procédé selon la revendication 1, dans lequel la matière polymérisable comprend un composé acrylique polymérisable et un système d'initiation de la polymérisation capable d'être activé par la lumière.

3. Procédé selon la revendication 2, dans lequel la matière polymérisable comprend un mélange d'oligomères et de monomères acryliques polymérisables.

4. Procédé selon la revendication 2, dans lequel la matière polymérisable comprend en outre une charge particulaire, la charge particulaire étant de préférence choisie dans le groupe constitué par la silice, l'alumine, le nitrure de silicium, et les composés de verre.

5. Procédé selon la revendication 2, dans lequel le système d'initiation de la polymérisation comprend un agent photoactif choisi dans le groupe constitué par la camphrequinone ; l'oxyde de 2,4,6-triméthylbenzoyldiphénylphosphine ; et le (4-N,N-diméthylamino)benzoate d'éthyle.

6. Procédé selon la revendication 2, dans lequel la matière polymérisable comprend en outre un pigment, le pigment étant de préférence choisi parmi les pigments organiques et inorganiques, ou le pigment étant de préférence choisi dans le groupe constitué par l'oxyde de fer noir 7053, l'oxyde de fer jaune 7055, le dioxyde de titane, le rouge Cromophtal-BRN 2-naphtalènecarboxamide, le N,N'-(2-chloro-1,4-phénylène)bis{4-{(2,5-dichlorophényl)-azo}-3-hydroxy}, le bleu marine, l'oxyde de fer brun 420, et leurs mélanges.

7. Procédé selon la revendication 1, dans lequel la prothèse est exposée à une lumière visible bleue ayant une longueur d'onde dans la plage d'environ 400 à environ 500 nm, ou dans lequel le patient mord dans la prothèse non durcie avant son retrait de la cavité buccale de façon que l'ajustement de la prothèse puisse être vérifié, ou dans lequel un matériau de séparation est projeté à partir de l'imprimante à jet d'encre et déposé entre le matériau de support et la matière polymérisable de type cire.

8. Procédé de fabrication d'une prothèse dentaire tridimensionnelle contenant de multiples couches transversales, comprenant les étapes suivantes :
a. application d'une matière polymérisable de type cire dans un lit de poudre comprenant un matériau pulvérulent particulaire pour former une prothèse multicouche, non durcie, chaque couche de la prothèse étant formée par projection par une imprimante à jet d'encre de la matière polymérisable dans une couche de la poudre particulaire de façon que les particules se lient dans des zones choisies ;
b. positionnement de la prothèse dans la cavité buccale d'un patient et façonnage de la prothèse sur une zone ciblée par application d'une pression suffisante ;
c. retrait de la prothèse façonnée de la cavité buccale ;
d. exposition de la prothèse à une lumière de durcissement de façon que la prothèse se solidifie complètement et durcisse.

9. Procédé selon la revendication 8, dans lequel la matière polymérisable comprend un composé acrylique polymérisable et un système d'initiation de la polymérisation capable d'être activé par la lumière.

10. Procédé selon la revendication 9, dans lequel la matière polymérisable comprend un mélange d'oligomères et de monomères acryliques polymérisables.

11. Procédé selon la revendication 8, dans lequel le matériau de lit de poudre comprend une poudre particulaire ayant un point de fusion supérieur à la matière polymérisable de type cire projetée.

12. Procédé selon la revendication 11, dans lequel la poudre particulaire comprend en outre un pigment.

13. Procédé selon la revendication 12, dans lequel le pigment est choisi parmi les pigments organiques et inorganiques.

14. Procédé selon la revendication 8, dans lequel la matière polymérisable de type cire comprend en outre un pigment, dans lequel le pigment est choisi parmi les pigments organiques et inorganiques.
